(19) Europäisches Patentamt
European Patent Office
Office européen des brevets

(11) **EP 4 279 455 A1**

(12) **EUROPEAN PATENT APPLICATION**
published in accordance with Art. 153(4) EPC

(43) Date of publication:
**22.11.2023 Bulletin 2023/47**

(21) Application number: **22739419.4**

(22) Date of filing: **12.01.2022**

(51) International Patent Classification (IPC):
***C01B 33/18*** *(2006.01)*

(52) Cooperative Patent Classification (CPC):
**C01B 33/18**

(86) International application number:
**PCT/JP2022/000754**

(87) International publication number:
**WO 2022/154014 (21.07.2022 Gazette 2022/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.01.2021 JP 2021003912**

(71) Applicant: **Tokuyama Corporation
Shunan-shi, Yamaguchi 745-8648 (JP)**

(72) Inventors:
• **YOSHIMURA, Noriko
Shunan-shi, Yamaguchi 745-8648 (JP)**
• **FUKUJU, Tadahiro
Shunan-shi, Yamaguchi 745-8648 (JP)**

(74) Representative: **Viering, Jentschura & Partner
mbB
Patent- und Rechtsanwälte
Am Brauhaus 8
01099 Dresden (DE)**

(54) **POROUS SPHERICAL SILICA AND METHOD FOR PRODUCING SAME**

(57) Provide is a porous spherical silica useful as a polish and as a cosmetic material. The porous spherical silica is such that less impurities are contained therein (the alkali metal content thereof is low), the particle size distribution thereof is narrow, the D50 (50% cumulative diameter of the volume based particle size distribution) thereof is within a predetermined range, and the pore volume thereof is within a predetermined range. The porous spherical silica is such that the D50 thereof by a laser diffraction scattering method is 2 to 200 μm, the D10/D90 thereof is at least 0.3, the pore volume thereof is 0.5 mL/g to 8 mL/g, the arithmetic mean value of the breaking compressive test forces of specimens of ten particles is $1.0 \times 10^1$ to $1.0 \times 10^2$ mN, the compressive test forces being obtained when the loading speed is 38.7363 mN/sec, and the alkali metal content thereof is at most 50 ppm. The porous spherical silica can be produced by forming a W/O emulsion using a fumed silica dispersion as an aqueous phase, and pH-adjusting or heating to gelate this emulsion, and thereafter collecting and drying the resultant.

EP 4 279 455 A1

**Description**

[Technical Field]

The present invention relates to a novel porous spherical silica and to a method of producing this novel porous spherical silica.

[Background Art]

Porous silica has been researched in a variety of ways, and porous silicas having a variety of physical properties are proposed. For example, a porous silica is produced by the method of: adding a mineral acid to an aqueous solution of an alkali metal silicate and thereby neutralizing this solution; and separating and collecting generated particles (patent literatures 1 and 2). The porous spherical silica obtained by such a method has the features such as a large pore volume and a narrow particle size distribution. Particularly, when such a porous spherical silica is used as a polish for industrial products and the like, the permeation of the resin on a polishing pad into the pores makes it easy to fix the porous spherical silica to the resin. However, because using an aqueous solution of an alkali metal silicate as a raw material, such a porous spherical silica contains impurities such as sodium, which is problematic. It is difficult to use such a porous spherical silica as a polish for products that do not like alkali metals, such as semiconductors.
Patent literature 3 proposes the method of spray-drying a fumed silica dispersion to obtain a porous spherical silica with a reduced alkali metal content. However, because the porous spherical silica obtained by the method of patent literature 3 is formed by spray-drying, the particle size distribution thereof is broad, which is problematic.

[Citation List]

[Patent Literature]

Patent Literature 1: WO 2004/101139 A1
Patent Literature 2: WO 2012/057086 A1
Patent Literature 3: WO 2019/131873 A1

[Summary of Invention]

[Technical Problem]

Therefore, an object of the present invention is to provide: a porous spherical silica such that the alkali metal content thereof is low, the particle size distribution thereof is narrow, the D50 (50% cumulative diameter of the volume based particle size distribution) thereof is within a predetermined range, and the pore volume thereof is within a predetermined range; and a method of producing such a porous spherical silica.

[Solution to Problem]

As a result of the intensive research of the inventors of the present invention for solving the aforementioned problems, the inventors found out that a porous spherical silica such that the alkali metal content thereof is reduced, the particle size distribution thereof is narrow, the D50 (50% cumulative diameter of the volume based particle size distribution) thereof is within a predetermined range, and the pore volume thereof is within a predetermined range can be produced by, in the step of producing the porous spherical silica, forming a fumed silica dispersion by an emulsion method, and thereafter, turning into a gel, which led to the completion of the present invention.
That is, the present invention is provided with a porous spherical silica characterized in that a 50% cumulative diameter (D50) of volume based particle size distribution measured by a laser diffraction scattering method ranges from 2 to 200 $\mu$m, a ratio (D10/D90) of a 10% cumulative diameter (D10) of the distribution to a 90% cumulative diameter (D90) of the distribution is at least 0.3, a pore volume by a BJH method is 0.5 mL/g to 8 mL/g, an arithmetic mean value of "compressive test forces when specimens are found to break" is $1.0 \times 10^1$ to $1.0 \times 10^2$ mN, the specimens being ten of the particles, the compressive test forces being obtained according to a method specified in JIS Z8844:2019 when a loading speed is 38.7363 mN/sec, and an alkali metal content is at most 50 ppm.
The present invention is provided with a porous spherical silica characterized in that a 50% cumulative diameter (D50) of volume based particle size distribution measured by a laser diffraction scattering method ranges from 2 to 200

μm, a ratio (D10/D90) of a 10% cumulative diameter (D10) of the distribution to a 90% cumulative diameter (D90) of the distribution is at least 0.3, a pore volume by a BJH method is 0.5 mL/g to 8 mL/g, an arithmetic mean value of "compressive test forces when specimens are found to break" is $1.0 \times 10^{-1}$ to $1.0 \times 10^1$ mN, the specimens being ten of the particles, the compressive test forces being obtained according to a method specified in JIS Z8844 when a loading speed is 0.4462 mN/sec, and an alkali metal content is at most 50 ppm.

The present invention is provided with a porous spherical silica characterized in that a 50% cumulative diameter (D50) of volume based particle size distribution measured by a laser diffraction scattering method ranges from 2 to 200 μm, a ratio (D10/D90) of a 10% cumulative diameter (D10) of the distribution to a 90% cumulative diameter (D90) of the distribution is at least 0.3, a pore volume by a BJH method is 0.5 mL/g to 8 mL/g, a mode pore radius by a BJH method is 5 nm to 50 nm, a specific surface area by a BET method is 100 $m^2$/g to 400 $m^2$/g, and an alkali metal content is at most 50 ppm.

The porous spherical silica can be produced by preparing a W/O emulsion comprising an aqueous phase where a fumed silica is dispersed, and an organic phase including a nonaqueous solvent as a major component; and next, heating the W/O emulsion to gelate the aqueous phase to obtain a porous spherical silica dispersion; and thereafter, collecting the generated porous spherical silica from the dispersion.

[Advantageous Effects of Invention]

The porous spherical silica according to the present invention is such that the particle size distribution thereof is narrow as shown by the high D10/D90, the alkali metal content thereof is low, the D50 (50% cumulative diameter of the volume based particle size distribution) thereof is within a predetermined range, and the pore volume thereof is within a predetermined range, which allow precise polishing; and is extremely useful as a polish for products that do not like alkali metals to be contained therein, such as semiconductors. Further, this porous spherical silica can give a smooth feel when used as an additive for cosmetics.

In the producing method according to the present invention, a high-purity fumed silica can be used as a raw material, and appropriate selection of the specific surface area of the fumed silica enables the specific surface area of the porous silica to be obtained to be arbitrarily controlled. This producing method also enables a porous spherical silica of a large pore volume to be obtained without any surface treatment since fumed silica, which is used as a raw material, itself has an agglomerating structure, which suppresses a decrease in pore volume due to drying shrinkage. Therefore, this producing method has many advantages as the method of producing a high-purity porous spherical silica with no alkali metal content.

[Description of Embodiments]

The following embodiments are examples of the present invention. The present invention is not limited to these embodiments.

<Porous Spherical Silica>

The porous spherical silica according to the present invention is such that the 50% cumulative diameter (D50) of the volume based particle size distribution which is measured by a laser diffraction scattering method of measurement ranges from 2 to 200 μm, and the ratio (D10/D90) of the 10% cumulative diameter (D10) of the aforementioned distribution to the 90% cumulative diameter (D90) of the aforementioned distribution is at least 0.3. A porous spherical silica having a diameter in these ranges is particularly suitably used as a polish or a cosmetic material. This D50 preferably ranges from 2 to 100 μm, particularly preferably from 5 to 50 μm, and further preferably from 5 to 20 μm. This D10/D90 is preferably at least 0.4, and further preferably at least 0.5. It is impossible that the D10/D90 exceeds 1.0. Generally, the D10/D90 is at most 0.6.

The pore volume of the porous spherical silica according to the present invention, which is measured by the following BJH method, is 0.5 ml/g to 8 ml/g. It is difficult to obtain a porous spherical silica of a large pore volume exceeding 8 ml/g. A porous spherical silica of a pore volume of at most 6 ml/g is easier, that of at most 4 ml/g is much easier, and that of at most 2.5 ml/g is particularly easier to produce. In particular, in order for the porous spherical silica according to the present invention to have good oil absorption characteristics, the pore volume thereof is preferably at least 1.0 ml/g, further preferably at least 1.6 ml/g, and more preferably at least 2.0 ml/g. A porous spherical silica having such a pore volume can be preferably used particularly as an additive for cosmetics.

The mode pore radius is preferably at least 5 nm, more preferably at least 10 nm, and further preferably at least 15 nm. The upper limit of this mode pore radius is preferably at most 50 nm, and more preferably at most 30 nm.

The pore volume and pore radius by the BJH method are obtained by drying a sample to be measured in a vacuum at 1 kPa or lower at 200°C for at least 3 hours, and thereafter, obtaining and analyzing only an adsorption isotherm in the adsorption of nitrogen at the liquid-nitrogen temperature by the BJH method (BARRETT, E. P., JOYNER, L. G., HALENDA, P. P. J. Am. Chem. Soc. 73,373 (1951). "The mode pore radius by the BJH method" means the value of the pore radius when a pore distribution curve (volume distribution curve) takes the maximum value: the pore distribution curve is plotted as the differential of the cumulative pore volume by the logarithm of the pore radius, which is obtained by the analysis by the BJH method, is on the vertical axis, and the pore radius is on the horizontal axis.

The specific surface area of the porous spherical silica according to the present invention by the BET method is 100 m$^2$/g to 400 m$^2$/g, is preferably at least 150 m$^2$/g, and particularly preferably ranges from 200 m$^2$/g to 350 m$^2$/g. The specific surface area of the porous spherical silica obtained by the producing method according to the present invention is the value obtained by subtracting several ten square meters per gram from the specific surface area of a fumed silica used as the raw material. When a fumed silica to be used as the raw material is selected so that a porous spherical silica having a specific surface area in the aforementioned range can be obtained, turning into a gel becomes easy, and the formation into a sphere becomes easy. Generally, the specific surface area of fumed silica is at most 400 m$^2$/g. Thus, it is difficult to obtain a porous spherical silica having a specific surface area exceeding 400 m$^2$/g. It is noted that the specific surface area is the value by the BET multipoint method using nitrogen adsorption.

Preferably, the arithmetic mean value of "compressive test forces when specimens are found to break" (hereinafter, "breaking compressive test forces") is $1.0 \times 10^1$ to $1.0 \times 10^2$ mN, where the specimens are ten particles of the porous spherical silica according to the present invention, and the compressive test forces are obtained according to the method specified in JIS Z8844:2019 when the loading speed is 38.7363 mN/sec. This arithmetic mean value is more preferably at most $5.0 \times 10^1$ mN, and further preferably at most $3.0 \times 10^1$ mN. The lower limit of this arithmetic mean value is more preferably at least $1.2 \times 10^1$ mN. Particles such that the breaking compressive test forces thereof lead to the arithmetic mean value within the aforementioned range are fragile under a pressure. In other words, the particles break when a specific pressure is applied thereto. Particularly, when the particles are used as a polish for industrial products and the like, the polished products are hardly scratched, and when used as a scrubbing material for cosmetic materials, the particles can reduce the irritation to the skin. A porous spherical silica such that the breaking compressive test forces lead to the arithmetic mean value lower than the aforementioned lower limit easily breaks, which significantly deteriorates workability. In contrast, a porous spherical silica such that the breaking compressive test forces lead to the arithmetic mean value exceeding the aforementioned upper limit does not break even when a strong force is applied thereto, so that any advantages as described above cannot be obtained.

Preferably, the arithmetic mean value of "compressive test forces when specimens are found to break" (hereinafter, "breaking compressive test forces") is $1.0 \times 10^{-1}$ to $1.0 \times 10^1$ mN, where the specimens are ten particles of the porous spherical silica according to the present invention, and the compressive test forces are obtained according to the method specified in JIS Z8844:2019 when the loading speed is 0.4462 mN/sec. This arithmetic mean value is more preferably at most $8.0 \times 10^0$ mN, and further preferably at most $5.0 \times 10^0$ mN. The lower limit of this arithmetic mean value is more preferably at least $3.0x\ 10^{-1}$ mN, and further preferably at least $6.0 \times\ 10^{-1}$ mN. Particles such that the breaking compressive test forces thereof lead to the arithmetic mean value within the aforementioned range are fragile under a pressure. In other words, the particles break when a specific pressure is applied thereto. Particularly, when the particles are used as a polish for industrial products and the like, the polished products are hardly scratched, and when used as a scrubbing material for cosmetic materials, the particles can reduce the irritation to the skin. A porous spherical silica such that the breaking compressive test forces lead to the arithmetic mean value lower than the aforementioned lower limit easily breaks, which significantly deteriorates workability. In contrast, a porous spherical silica such that the breaking compressive test forces lead to the arithmetic mean value exceeding the aforementioned upper limit does not break even when a strong force is applied thereto, so that any advantages as described above cannot be obtained.

The alkali metal content of the porous spherical silica according to the present invention is at most 50 ppm (on the mass basis), particularly preferably at most 30 ppm, and more preferably at most 10 ppm. Such a porous spherical silica can be preferably used as a polish for materials for semiconductor substrates in particular.

The porous silica according to the present invention has a spherical shape. Here, the spherical shape means that the average circularity obtained by image analysis using a Scanning Electron Microscope (SEM) is at least 0.8. "Average circularity obtained by image analysis" is the value of the arithmetic mean value of the circularity obtained by the image analysis of a SEM image of at least 2000 particles of the porous spherical silica which is observed with a SEM at the magnification of 1000. Here, "circularity" is the value calculated by the following equation (1):

$$(1): C = 4\pi S/L^2$$

In the equation (1), C means the circularity, S means the area of the porous spherical silica in the image (project area), and L means the length of the circumference (perimeter) of the porous spherical silica in the image. This circularity is particularly preferably at least 0.85. Normally, there is no corner in the particle image of the porous spherical silica according to the present invention which is obtained by SEM observation at the magnification of 1000.

The porous spherical silica according to the present invention may be hydrophilic and may be hydrophobic. The hydrophilic porous spherical silica can be produced by the producing method described later. The hydrophobic porous spherical silica can be obtained by, after the gelating step in the producing method, undergoing treatment by adding a surface-treatment agent to the reaction system before the step of collecting gels; or drying the gels to obtain the hydrophilic porous spherical silica, and thereafter, appropriately applying a method of surface-treating. "Hydrophilic" as used herein means being dispersible in water containing no organic solvent.

The porous spherical silica according to the present invention has the aforementioned characteristics, and thus, can be preferably used as a polish, a cosmetic material, and the like. When this porous spherical silica is used as a polish, the polishing method is not particularly limited. This porous spherical silica can be used for any of dry polishing and wet polishing. When the porous spherical silica according to the present invention is fixed to a resin pad, to be used as a polishing wheel, the permeation of the resin into the pores makes it easy to fix the porous spherical silica. When used as a cosmetic material, this porous spherical silica can be used as an additive for cosmetics such as foundation, or a scrubbing material because of good oil absorption characteristics derived from the porosity thereof, and a smooth feel derived from the spherical shape thereof.

<Method of Producing Porous Spherical Silica>

The method of producing the porous spherical silica according to the present invention is not particular limited. The aforementioned large pore volume and large modal diameter of the pore radius are easily realized by the use of a fumed silica dispersion as a raw material. In general, fumed silica has the structure of aggregating fine particulate silica (primary particles). Therefore, it suppresses a decrease in pore volume due to drying shrinkage that, by the use of a fumed silica dispersion as a raw material of the porous spherical silica, the fumed silica in the dispersion is gelated to form a network. This allows a porous spherical silica having a large pore volume to be obtained without any surface treatment.

More specifically, the method of producing the porous spherical silica comprises: preparing a W/O emulsion comprising an aqueous phase where the fumed silica is dispersed and an organic phase that includes a nonaqueous solvent as the major component (step of preparing a W/O emulsion); next, heating the emulsion to gelate the aqueous phase to obtain a porous spherical silica dispersion (gelating step); and thereafter, collecting the generated porous spherical silica from the dispersion (step of collecting gels). Hereinafter, each of the steps will be described in detail.

(Step of Preparing W/O Emulsion)

As the step of preparing a W/O emulsion comprising an aqueous phase where the fumed silica is dispersed and an organic phase that includes a nonaqueous solvent as the major component, the following steps are particularly preferably employed: first, a dispersion where the fumed silica is dispersed in a water phase is prepared (step of preparing a dispersion), and using this dispersion and an organic solvent, an emulsion is prepared according to a usual way (step of forming emulsion). These steps will be further described below.

(Step of Preparing Dispersion)

The step of preparing a dispersion is the step of dispersing the fumed silica in water to prepare a dispersion.

The fumed silica used herein is dispersible in water, and can be gelated by heating, adjusting pH, etc. A silica having a large number of silanol groups on the surface thereof also has such properties. Thus, what is called fumed silicas without surface treatment can be mostly used. Because of easy progress of the gelation, a fumed silica having a specific surface area of at least 100 $m^2/g$, particularly at least 200 $m^2/g$ is preferably used. This specific surface area is further preferably at least 250 $m^2/g$. The larger the specific surface area is, the faster the speed at which the gelation progresses is, and the easier it is to gelate the droplets where the fumed silica is dispersed (W-phase). Because of availability, a fumed silica having a specific surface area of 400 $m^2/g$ as the upper limit is preferably used. It is noted that the specific surface area is the value by the BET multipoint method using nitrogen adsorption.

The specific surface area of the porous spherical silica obtained by the method described here is the value obtained by subtracting several ten square meters per gram from the specific surface area of the fumed silica used as a raw material. Thus, the specific surface area of the porous spherical silica can be controlled arbitrarily by appropriately selecting a fumed silica to be used as the raw material according to the specific surface area of the porous spherical silica to be aimed without any change in the producing conditions. Fumed silicas having different specific surface areas can be used in combination as the fumed silica used in the present invention.

Fumed silicas as described above are commercially available. For example, any of the following can be used: various hydrophilic grades of REOLOSIL from Tokuyama Corporation, various hydrophilic grades of AEROSIL from NIPPON AEROSIL CO., LTD., and various hydrophilic grades of dry silica HDK from Wacker Asahikasei Silicone Co., Ltd.

In general, fumed silica is of high purity, and contains almost no impurities such as alkali metals. Thus, the alkali metal content of the produced porous spherical silica can be extremely low.

Water is essential as a solvent in this step. Any other solvent may be contained as long as not hindering the formation of the emulsion, or the subsequent gelation. When a latent base is used for promoting the undermentioned gelation, dissolution in water before the fumed silica is dispersed is recommended.

As the method of dispersing the fumed silica in the solvent, preferably, a dispersion where the fumed silica is preliminarily dispersed in the solvent is prepared, and fine dispersion is carried out with a disintegrator or the like. Specific examples of a disintegrator that can be used for the fine dispersion include a ball mill, a bead mill, a vibration mill, a pin mill, an atomizer, a colloid mill, a homogenizer, a high pressure homogenizer, and an ultrasonic homogenizer. As the extent of the dispersion as a result of the fine dispersion, preferably, the value of the D90 is at most 0.5 $\mu$m when the particle size distribution of the dispersion is measured by a laser diffraction scattering method.

The silica concentration of the fumed silica dispersion preferably ranges from 10wt% to 30wt%, is more preferably at least 15wt%, and is particularly preferably at least 20wt%. The higher the silica concentration of the fumed silica dispersion is, the faster the speed at which the gelation progresses is. Too high a silica concentration of the fumed silica dispersion, however, leads to the loss of the fluidity, which makes it difficult to form the fumed silica dispersion.

The gelation of the fumed silica dispersion is accelerated by heating. The progress of the gelation of the fumed silica dispersion at the step of preparing a dispersion makes it difficult for the W-phase to form spheres in the subsequent step of forming emulsion, or in an extreme case, makes it difficult to form the emulsion itself. Therefore, in the step of preparing a dispersion, the temperature of the fumed silica dispersion is preferably kept at approximately room temperature (20°C) or lower. It is also effective to cool the fumed silica dispersion to a temperature lower than room temperature (preferably at most 15°C, more preferably at most 12°C) when the specific surface area of the fumed silica is large and/or the concentration thereof is high, and thus, the gelation easily progresses.

(Step of Forming Emulsion)

The step of preparing a W/O emulsion is the step of dispersing, in the nonaqueous solvent, the fumed silica dispersion obtained in the step of preparing a dispersion to form the W/O emulsion. The formation of such a W/O emulsion causes the fumed silica dispersion, which is a dispersoid, to form spheres due to surface tension etc. Thus, the gelation of the fumed silica dispersion dispersed in the nonaqueous solvent in the form of sphere allows spherical gels to be obtained.

A solvent having hydrophobicity such that the emulsion can be formed with the fumed silica dispersion may be used as the nonaqueous solvent used in the producing method according to the present invention. As such a solvent, for example, an organic solvent of any of hydrocarbons, halogenated hydrocarbons, and the like can be used. More specific examples of such a solvent include nonaqueous solvents of any of hexane, heptane, octane, nonane, decane, liquid paraffin, dichloromethane, chloroform, carbon tetrachloride, and dichloropropane. Among them, any of hexane, heptane, and decane, which have moderate viscosities, can be preferably used. If necessary, a plurality of the solvents may be used in combination. Any hydrophilic solvent such as lower alcohols can be also used in combination (used as a mixed solvent) as long as the emulsion can be formed together with the fumed silica dispersion.

The used amount of the nonaqueous solvent is not particularly limited as long as the W/O emulsion can be formed. Generally, approximately 1 to 10 parts by volume of the nonaqueous solvent is used per 1 part by volume of the fumed silica dispersion.

In this producing method, a surfactant is preferably added when the W/O emulsion is formed. As the surfactant used herein, any of known surfactants that are used for forming W/O emulsions can be used without limitations, and any of anionic surfactants, cationic surfactants, and nonionic surfactants can be used. Among them, nonionic surfactants are preferrable because the W/O emulsion is easily generated, and an alkali metal is hardly contained. In particular, a surfactant having a HLB value of 3 to 5 can be preferably used: the HLB value indicates the degree of the hydrophilicity or hydrophobicity of the surfactant, and here, means the HLB value according to Griffin's method. Specific examples of surfactants which can be preferably used here include sorbitan monooleate, sorbitan monostearate, and sorbitan monosesquioleate.

The used amount of the surfactant does not change from the usual amount when a W/O emulsion is formed. Specifically, any amount in the range of 0.05 g to 10 g can be preferably used per 100 mL of the fumed silica dispersion.

As the method of dispersing the fumed silica dispersion in the nonaqueous solvent when the W/O emulsion is formed, any known method of forming a W/O emulsion can be used. In view of, for example, easy industrial production, the emulsion is preferably formed by mechanical emulsification: specific examples include the methods with a mixer, a homogenizer, and the like. Preferably, a homogenizer can be used. Through this step of forming emulsion, an emulsion such that the particle size distribution of the droplets in the aqueous phase is sharp is obtained. Thus, the particle size distribution of the finally obtained spherical porous silica is also sharp.

(Gelating Step)

The gelating step follows the step of preparing a W/O emulsion, and is the step of gelating the fumed silica dispersion in a state where the droplets of the fumed silica dispersion are dispersed in the nonaqueous solvent. This gelation can be carried out by a known method. For example, the gelation can be easily progressed by the technique of heating to a high temperature, or the technique of adjusting the pH of the fumed silica dispersion to be weakly acidic or basic. These techniques are preferable because the reaction thereby can be actively controlled. It is noted that the pH of a fumed silica dispersion prepared in the aforementioned steps without pH adjustment generally ranges from 3.0 to 4.5.

The heating is performed, so that the temperature should not exceed the boiling point of each of the used solvents. The lower limit of the gelating temperature is preferably 50°C, and more preferably 60°C; and the upper limit thereof is preferably at most 100°C, and more preferably at most 90°C.

The aforementioned pH adjustment can be easily performed by: the method of mixing, with the fumed silica dispersion, a substance that thermally decomposes by heating so as to show basicity (which shall be referred to as "latent base"), such as urea in advance, and perform heating in the gelation to raise the pH; or the method of, while stirring with, for example, a mixer to maintain the state where the W/O emulsion is formed, adding a base into the emulsion.

Specific examples of this base include: ammonia; tetraalkylammonium hydroxides such as tetramethylammonium hydroxide (TMAH); amines such as trimethylamine; alkali hydroxides such as sodium hydroxide; alkali metal carbonates such as sodium carbonate, and sodium hydrogen carbonate; and alkali metal silicates. The aforementioned stirring may be strong enough for the W/O emulsion and the base to be mixed with each other.

Among them, the method by the thermal decomposition of a latent base such as urea, or the use of any of ammonia, tetraalkylammonium hydroxides, and amines is preferable because no metal element is contained. When used for the pH adjustment, ammonia may be blown in as a gas, or may be added as an aqueous ammonia. It is particularly preferable to employ the pH adjustment using urea because the pH can be uniformly adjusted as a whole by heating.

As the pH when the pH is adjusted to promote the gelation, it is particularly preferable to adjust the amount of the addition, so that the value of the pH of the fumed silica dispersion rises to approximately 4.5 to 8.0. The following are specific amounts of the addition of urea to the fumed silica dispersion, which are also same of the latent base: preferably at least 1wt% and particularly preferably at least 2wt%; and as the upper limit, preferably at most 7wt% and more preferably at most 5wt%.

The compressive strength of the porous spherical silica can be controlled by adjusting the gelating conditions and the gelating time. The higher the gelating temperature is, and the longer the gelating time is, the more the gelation progresses, and the more firmly the aggregates of fine particulate silica (primary particles) is, and thus, the more the compressive strength of the porous spherical silica is.

Stirring when the heating or pH adjustment is performed is preferable in order to prevent the gels from agglomerating with each other. A known method is generally used for the stirring. For example, specifically, a mixer with a stirring blade can be used.

The state of the fumed silica dispersion changes from a liquid to a solid after the gelation. Thus, the system is not the W/O emulsion, but a dispersion (suspension) where solids (gels) are dispersed in a hydrophobic solvent.

(Step of Collecting Gels)

In this producing method, the gels generated as described above are collected from the dispersion. As the method of collecting the gels, any of general methods of solid-liquid separation, such as filtration and centrifugation, can be used. Prior to this collection, WO phase separation may be performed. The WO phase separation is to separate the dispersion of the gels into two phases of an O-phase and the W-phase, and is the operation generally called demulsification. Here, the gels obtained in the gelating step is present on the separated W-phase side. The separation of the W-phase from the O-phase makes it easy to collect the gels through solid-liquid separation by filtration or the like.

The method of performing the WO phase separation can be carried out by appropriately selecting a known method as the demulsifying method. Preferably, a certain amount of an aqueous organic solvent that is commonly used in demulsification is added to the dispersion of the gels, and the resultant is heated to be separated into the O-phase and the W-phase. After this step, generally, the upper layer is the O-phase (phase mainly including the organic solvent), and the lower layer is the W-phase (aqueous phase including the aqueous organic solvent and the gels).

Examples of the aqueous organic solvent include acetone, methanol, ethanol, and isopropyl alcohol. Among them, isopropyl alcohol can be particularly preferably used.

Preferably, the amount of the addition of the aqueous organic solvent is adjusted according to the type and the amount of the surfactant having a HLB of 3 to 5, which was used when the emulsion was formed. For example, when sorbitan monooleate was used as the surfactant, the demulsification can be preferably performed by adding the aqueous organic solvent approximately 1/6 to 1/2 times as much as the non-aqueous organic solvent (aqueous organic solvent/non-aqueous organic solvent) by mass, stirring the resultant if necessary, and thereafter, allowing the resultant to stand.

In the WO phase separation, the surfactant moves to the O-phase side (extraction). Thus, a porous spherical silica that contains no impurities from the surfactant can be obtained by removing the O-phase.

The temperature for the heating is at least 50°C, preferably ranges approximately from 50 to 80°C, and more preferably ranges approximately from 60 to 70°C.

After the aqueous organic solvent is added to the dispersion of the gels as described above, it is preferable to stir the resultant in order to prevent the gels from agglomerating with each other. Generally, a known method is used for the stirring. Specifically, for example, a mixer with a stirring blade can be used. The extent of the mixing is not particularly limited, but may be such that the stirring rotates the level of the dispersion, which is, for example, in the case of the stirring with the mixer, 0.1 to 3.0 kW/m$^3$, preferably 0.5 to 1.5 kW/m$^3$. As the stirring time, approximately 0.5 to 24 hours, preferably approximately 0.5 to 1 hour are proper.

After the WO phase separation, the W-phase including the gels are collected. Specifically, the O-phase (upper layer) can be separated and removed by, for example, decantation.

The porous spherical silica according to the present invention can be obtained by collecting the gels included in the collected W-phase by solid-liquid separation, and drying the collected gels. A general drying method can be used. In particular, for suppressing the agglomeration of the particles, it is preferable to employ a fluidized drying method, specifically, vibration-drying, flash-drying, spray-drying, or the like. The agglomeration of the particles can be also suppressed by, prior to the drying, solvent replacement of an organic solvent having a low surface tension, or a rinse of such an organic solvent for a cake after the solid-liquid separation. This organic solvent is preferably aqueous because the replacement for water remaining inside the pores becomes easy. Specific examples of an aqueous organic solvent herein include acetone, methanol, ethanol, and isopropyl alcohol.

The solvent replacement or rinse can also adjust the drying shrinkage inside the pores. Thus, the pore volume can be controlled by moderate drying shrinkage. When the solvent replacement is performed, lowering the concentration of the aqueous organic solvent to raise the ratio of the water more easily leads to the drying shrinkage, so that the pore volume is smaller. On the contrary, raising the concentration of the aqueous organic solvent leads to the suppression of the drying shrinkage, so that the pore volume is larger. When the rinse is given, reducing the amount of the used aqueous organic solvent causes the pore volume to be smaller.

The temperature in the drying is preferably at least the highest boiling point in the boiling points of all the solvents used in the period from the preparation of the fumed silica dispersion to the drying. The drying is preferably performed under atmospheric or reduced pressure. "At least the ... boiling point" means at least the boiling point of the solvent under the pressure in the drying.

Further, the porous spherical silica according to the present invention may be fired after the drying. Organic substances can be removed, and the breaking compressive test force can be adjusted by this firing. When the firing is performed for the purpose of removing organic substances, the firing temperature may be at least the boiling point of an organic substance to be removed which is used in the producing method according to the present invention. When the firing is performed for the purpose of adjusting the breaking compressive test force, the firing conditions may be adjusted so that the value to be aimed can be obtained. Generally, the longer the firing time is and the higher the firing temperature is, the stronger the breaking compressive test force is. Specifically, the breaking compressive test force can be increased by the firing at a temperature of 700 to 1000°C for approximately 10 hours without the agglomeration of the particles. The firing temperature is preferably at least 750°C, and more preferably 850°C. The firing time is preferably at least 8 hours, and more preferably at least 9 hours. The firing at a temperature of at least 1000°C leads to easy agglomeration of the particles.

Further, the dried porous spherical silica or the dried and fired porous spherical silica may be disintegrated. The disintegration can be carried out with a common disintegrator or the like. Specifically known is the processing method with a ball mill, a pin mill, a vibration mill, a bead mill, a jet mill, a masscolloider (trade name), or the like. Desirably, the disintegration conditions are arbitrarily adjusted according to the machine to be used. The conditions may be such that the particles are not broken but the agglomeration is disintegrated.

In the producing method, the particle diameter of the obtained porous spherical silica is substantially the same as the diameter of the droplet (W-phase) of the fumed silica dispersion in the W/O emulsion prepared in the step of forming emulsion. Therefore, it is necessary to set the dispersion conditions so that the particle diameter of the obtained porous spherical silica will be in the range of the aimed diameters. Various methods are known for controlling the diameter of the droplet in a W/O emulsion, and any techniques of them may be appropriately selected and applied. Any known method can be used for the method of adjusting the particle diameter of the droplet. Specific examples of such a method include the method of adjusting the amount of the addition of the surfactant, and the method of adjusting the shearing force applied in the emulsification according to the rotation speed, the flow rate, etc. In the adjustment of the amount of the addition of the surfactant, the larger the amount of the used surfactant is, the finer the droplets tend to be; and the smaller the amount thereof is, the larger the droplets tend to be. In the adjustment of the shearing force, the stronger the applied shearing force is, the finer the droplets tend to be; and the weaker the applied shearing force is, the larger the droplets tend to be.

The pore volume can be controlled by drying shrinkage. Any known method of controlling drying shrinkage can be used. Specific examples of such a method include the method by the aforementioned solvent replacement or rinse, or the like, and the method including an improved drying step such as freeze-drying and supercritical drying. The use of fumed silica as a raw material as in the producing method according to the present invention leads to a large the mode pore radius because fumed silica has an agglomerating structure. The specific surface area can be adjusted by appropriately selecting the specific surface area of the fumed silica used as a raw material, and optionally by the gelating time. It is noted that the shorter the gelating time is, the larger the specific surface area is.

The higher the gelating temperature in the gelating step is, the stronger the breaking compressive test force is. Conversely, the lower the gelating temperature is, the weaker the breaking compressive test force is. Or, the longer the gelating time is, the stronger the breaking compressive test force is. Conversely, the shorter the gelating time is, the weaker the breaking compressive test force is. The breaking compressive test force can be also controlled by the firing conditions. The longer the firing time is and the higher the firing temperature is, the stronger the breaking compressive test force is.

The alkali metal content can be easily lowered when a person skilled in the art takes enough care to avoid contamination (mixing with impurities) to perform the production using a fumed silica containing substantially no alkali metal as the raw material as described above, and other raw materials containing substantially no alkali metal. When further reduction of the alkali metal content is aimed, the cake may be washed with water, an organic solvent, or the like after the solid-liquid separation before the drying.

[Examples]

Hereinafter, examples for specifically describing the present invention will be shown. The present invention is not limited to these examples.

<Evaluation Method>

The produced porous spherical silicas were each evaluated concerning the following items.

(Measuring Particle Size Distribution by Laser Diffraction, and Volume-based Cumulative Diameter)

To 40 mL of an ion-exchanged water, 0.1 g of the porous spherical silica was added and dispersed therein using an ultrasonic cleaner (BRANSONIC 1510J-DTH manufactured by BRANSON) for 30 minutes. The particle size distribution of this dispersion was measured using LS 13 320 manufactured by Beckman Coulter. The refractive index of the solvent was 1.374, and the refractive index of the particles was 1.46. From the obtained particle size distribution, the 50%, 10%, and 90% cumulative diameters of the volume-based particle size distribution were evaluated.

(Measuring Pore Volume and Pore Radius (Mode Pore Radius) by BJH, and BET Specific Surface Area)

The pore volume and the pore radius (mode pore radius) by BJH, and the BET specific surface area were measured by BELSORP-mini (manufactured by BEL JAPAN, Inc.) according to the aforementioned definitions.

(Compressive Test Forces when Specimens are Found to Break)

"Compressive test forces when specimens are found to break" were measured by a Micro Compression Testing Machine (MCT-W510-J manufactured by Shimadzu Corporation) according to the aforementioned definitions. The measurement was performed under each of two conditions for the loading speed: 38.7363 mN/sec and 0.4462 mN/sec. The force-hold time was 10 seconds under the both conditions. For the measurement, an indenter of 200 $\mu$m in diameter was used.

(Alkali Metal Content)

To 1 g of the porous spherical silica, 10 mL of nitric acid, and 10 mL of hydrofluoric acid were added, so that the silica was dissolved therein. The resultant solution was heated at 180°C for 4 hours to evaporate to dryness. After the resultant was cooled to room temperature, 2 mL of nitric acid and 18 mL of ultrapure water were added thereto, so that 20 mL of a sample to be measured was obtained in a volumetric flask. The alkali metal content of the obtained sample was measured using an inductively coupled plasma-optical emission spectrometer (ICAP 650DUO manufactured by Thermo Scientific).

(Average Circularity)

A SEM image of at least 2000 particles of the porous spherical silica which was observed with a SEM (S-5500 manufactured by Hitachi High-Tech Corporation, 3.0 kV in acceleration voltage, secondary electron detection) at the magnification of 1000 was analyzed, and the average circularity was calculated according to the aforementioned definition.

<Example 1>

(Step of Preparing Dispersion)

To 200 mL of an ion-exchanged water where 6.65 g of urea was dissolved, 66 g of REOLOSII, QS-30 (manufactured by Tokuyama Corporation) was added while stirring the mixture by the use of a homogenizer (T25BS1 manufactured by IKA), so that a fumed silica was predispersed therein. Thereafter, the resultant was finely dispersed using an ultrasonic homogenizer (Sonifier SFX250 manufactured by BRANSON), so that a fumed silica dispersion was obtained. The particle size distribution of the dispersion as a result of the dispersing was measured by a laser diffraction scattering method. As a result, the value of the D90 was 0.19 $\mu$m. This step of preparing a dispersion was performed in a chiller that was cooled to 10°C.

(Step of Preparing W/O Emulsion)

From the fumed silica dispersion prepared by the foregoing step, 65.5 g of the dispersion was separated out. To the separated dispersion, 129 g of decane where 0.75 g of sorbitan monooleate (RHEODOL SP-O10V manufactured by Kao Corporation) was dispersed was added. Thereafter, the resultant was stirred using a homogenizer at 4000 rpm for 3 minutes, so that a W/O emulsion was obtained.

(Gelating Step)

The obtained W/O emulsion was kept in a water bath at 80°C for 3 hours while stirred at 300 rpm using four inclined-blades each having a blade diameter of 60 mm, a blade width of 20 mm, and an inclination of 45 degrees, so as to be gelated.

(Step of Collecting Gels)

To the resultant, 77 g of isopropyl alcohol and 52 g of water were added. The mixture was stirred with a stirring blade while kept at 70°C for 30 minutes. Thereafter, the resultant was allowed to stand so as to be separated into two layers of the upper layer, which was an O-phase, and the lower layer, which was a W-phase.
Next, by decantation, the O-phase and the W-phase were separated, and the W-phase was collected.
The obtained gels were filtered off from the W-phase by a suction filter. The collected gels were dried with a vacuum dryer at 150°C for 12 hours. The physical properties of the porous spherical silica that was obtained in this way are shown in table 1 (the physical properties of the porous spherical silicas obtained in the following examples and comparative examples are also shown in table 1).

<Example 2>

A porous spherical silica was obtained in the same process as in example 1 except that the rotation speed of the homogenizer in the step of preparing a W/O emulsion was changed to 8600 rpm, and except that after the drying, firing was performed at 800°C for 10 hours.

<Example 3>

A porous spherical silica was obtained in the same process as in example 2 except that the fumed silica as a raw material was changed from REOLOSII, QS-30 to REOLOSII, QS-40 (manufactured by Tokuyama Corporation), and except that the gelating time was 1 hour. The value of the D90 of the fumed silica dispersion after the step of preparing a dispersion was 0.15 $\mu$m.

<Example 4>

A porous spherical silica was obtained in the same process as in example 1 except that in the step of preparing a W/O emulsion, the homogenizer was changed to a stirring blade, and the rotation speed was 400 rpm and the stirring time was 1 hour.

<Example 5>

A porous spherical silica was obtained in the same process as in example 1 except that the rotation speed of the homogenizer in the step of preparing a W/O emulsion was changed to 1000 rpm, and except that after the drying, firing was performed at 900°C for 10 hours.

<Example 6>

A porous spherical silica was obtained in the same process as in example 2 except that the rotation speed of the homogenizer in the step of preparing a W/O emulsion was changed to 3000 rpm, and except that the cake was rinsed with 100 g of isopropyl alcohol before the drying.

<Example 7>

A porous spherical silica was obtained in the same process as in example 2 except that in the firing step, firing was performed at 600°C for 1 hour.

<Comparative Example 1>

A porous spherical silica was obtained by spray-drying a fumed silica dispersion prepared in the same process as in the step of preparing a dispersion in example 1, and thereafter, firing the resultant at 600°C for 1 hour.

<Comparative Example 2>

To 9 g/100 mL of an aqueous solution of sodium silicate (the molar ratio of $SiO_2Na_2O$ thereof was 3.1), 10 g/100 mL of sulfuric acid was added, so that the pH of the resultant was 2.9. Thereby, 500 mL of a silica sol was prepared. Gels were obtained through the step of preparing a W/O emulsion, the gelating step, and the step of collecting gels in the same process as in example 2 except that the fumed silica dispersion was changed to 66.5 g of the silica sol which was separated out from the prepared silica sol. The obtained gels were put in a pressure filter, and washed with water until the electric conductivity of the filtrate was at most 100 $\mu$S/cm. At this time, 5 L of an ion-exchanged water was necessary until the electric conductivity of the filtrate was the aforementioned value. A porous spherical silica was obtained by drying the washed gels with a vacuum dryer at 150°C for 12 hours.

<Comparative Example 3>

To a mixed solution of 6.4 mL of tetraethyl orthosilicate and 5.26 mL of ethanol, 10.8 mL of an ion-exchanged water such that the pH thereof was adjusted to 2 by hydrochloric acid was added. Thereby, a silica sol was prepared. At this time, the molar ratio of tetraethyl orthosilicate:ion-exchanged water: ethanol was 1:20:3. A porous spherical silica was obtained in the same process as in example 2 except that the fumed silica dispersion was changed to 66.5 g of the silica sol which was separated out from the prepared silica sol.

Table 1

| (Table 1) | D50 [μm] | D10/D90 | Pore volume [ml/g] | Mode pore radius [nm] | Specific surface area [m²/g] | Compressive test force [mN] (38.7363 mN/sec) | Compressive test force [mN] (0.4462 mN/sec) | Alkali metal content [ppm] | Average circularity |
|---|---|---|---|---|---|---|---|---|---|
| Example 1 | 15.1 | 0.46 | 2.1 | 25 | 255 | 14 | 0.42 | 9 | 0.88 |
| Example 2 | 10.2 | 0.60 | 1.4 | 18 | 243 | 14 | 3.4 | 16 | 0.90 |
| Example 3 | 11.4 | 0.55 | 1.6 | 18 | 326 | 18 | 3.8 | 13 | 0.84 |
| Example 4 | 110.9 | 0.57 | 1.4 | 16 | 225 | 13 | 2.1 | 11 | 0.87 |
| Example 5 | 5.0 | 0.52 | 2.1 | 22 | 275 | 19 | 4.0 | 19 | 0.85 |
| Example 6 | 20.0 | 0.49 | 1.5 | 24 | 269 | 13 | 2.5 | 25 | 0.84 |
| Example 7 | 10.0 | 0.55 | 1.8 | 18 | 231 | 13 | 1.0 | 12 | 0.88 |
| Comparative example 1 | 9.8 | 0.25 | 1.8 | 24 | 255 | 12 | 0.50 | 9 | 0.90 |
| Comparative example 2 | 10.5 | 0.55 | 0.4 | 18 | 654 | 25 | 5.5 | 155 | 0.86 |
| Comparative example 3 | 4.9 | 0.15 | 0.6 | not measured | 684 | not measured | not measured | 12 | 0.67 |

EP 4 279 455 A1

<Evaluation Results>

(Examples 1 to 7)

As shown in table 1, in each of examples 1 to 7, the porous spherical silica such that the D10/D90 was at least 0.3, that is, the particle size distribution thereof was narrow, and the alkali metal content was reduced to at most 50 ppm could be produced. These were derived from the use of fumed silica for the raw material, and the formation by an emulsion method as in the producing method according to the present invention. Each of all the porous spherical silica obtained in examples 1 to 7 was such that the D50 ranged from 2 to 200 $\mu$m, the pore volume by the BJH method ranged from 0.5 to 8 mL/g, the mode pore radius by the BJH method ranged from 5 nm to 50 nm, the specific surface area by the BET method ranged from 100 m$^2$/g to 400 m$^2$/g, the arithmetic mean value of "compressive test forces when specimens were found to break" ranged from $1.0 \times 10^1$ to $1.0 \times 10^2$ mN, where the specimens were ten of the particles, and the compressive test forces were obtained according to the method specified in JIS Z8844:2019 when the loading speed was 38.7363 mN/sec, and the arithmetic mean value of the "compressive test forces when specimens were found to break" when the loading speed was 0.4462 mN/sec ranged from $1.0 \times 10^{-1}$ to $1.0 \times 10^1$ mN, where the specimens were ten of the particles.
In examples 1 to 7, porous spherical silicas having various values of the D50 were obtained. All the values of the D50 were each controlled by adjusting the diameter of the droplet in the W/O emulsion by changing the rotation speed in the step of forming emulsion, and/or by changing emulsification equipment. In the producing method according to the present invention, the value of the D50 of the porous spherical silica can be easily controlled without any large change in producing process.

(Example 3)

The porous spherical silica in example 3 showed a specific surface area larger than example 1 because the fumed silica having a specific surface area larger than example 1 was used in example 3, and because a decrease in specific surface area due to the progress of the gelation (aging) was suppressed by shortening the gelating time. As described, the specific surface area can be arbitrarily adjusted by appropriately selecting a fumed silica to be used as the raw material, and adjusting the gelating time.

(Example 5)

The porous spherical silica in example 5 showed a larger value for the breaking compressive test force than example 1 or 2 because, in example 5, the firing step was added to the steps as in example 1 and the firing temperature was higher than example 2. As described, the breaking compressive test force can be controlled by adding the firing step, and/or adjusting the firing conditions in the firing step.

(Comparative Example 1)

The porous spherical silica in comparative example 1, which was formed by spray-drying, had the D10/D90 of less than 0.3, that is, a broad particle size distribution. As described, it is difficult to obtain a porous spherical silica having D10/D90 exceeding 0.3, that is, a narrow particle size distribution by spray-drying.

(Comparative Example 2)

The porous spherical silica in comparative example 2, which used sodium silicate as a raw material, showed a higher alkali metal content than any of examples 1 to 7. Such a higher alkali metal content is considered to be because sodium derived from sodium silicate remained in the porous spherical silica. In comparative example 2, after the step of collecting gels, the gels were sufficiently washed with the ion-exchanged water until the electric conductivity of the filtrate was at most 100 $\mu$S/cm. However, the alkali metal content could not be reduced. As described, when sodium silicate is used as a raw material, it is difficult to obtain a porous spherical silica having a reduced alkali metal content.

(Comparative Example 3)

The porous spherical silica in comparative example 3, which used tetraethyl orthosilicate as a raw material, had a circularity of less than 0.8. This is considered to be because ethanol was generated when the tetraethyl orthosilicate was gelated, which destabilized the emulsion to make it difficult to keep the spherical shape. Because of the foregoing characteristics, tetraethyl orthosilicate is unsuitable for formation into a spherical shape by an emulsion method.

**Claims**

1. A porous spherical silica **characterized in that**

   a 50% cumulative diameter (D50) of volume based particle size distribution measured by a laser diffraction scattering method ranges from 2 to 200 $\mu$m,
   a ratio (D10/D90) of a 10% cumulative diameter (D10) of the distribution to a 90% cumulative diameter (D90) of the distribution is at least 0.3,
   a pore volume by a BJH method is 0.5 mL/g to 8 mL/g,
   an arithmetic mean value of "compressive test forces when specimens are found to break" is $1.0 \times 10^1$ to $1.0 \times 10^2$ mN, the specimens being ten of the particles, the compressive test forces being obtained according to a method specified in JIS Z8844:2019 when a loading speed is 38.7363 mN/sec, and
   an alkali metal content is at most 50 ppm.

2. A porous spherical silica **characterized in that**

   a 50% cumulative diameter (D50) of volume based particle size distribution measured by a laser diffraction scattering method ranges from 2 to 200 $\mu$m,
   a ratio (D10/D90) of a 10% cumulative diameter (D10) of the distribution to a 90% cumulative diameter (D90) of the distribution is at least 0.3,
   a pore volume by a BJH method is 0.5 mL/g to 8 mL/g,
   an arithmetic mean value of "compressive test forces when specimens are found to break" is $1.0 \times 10^{-1}$ to $1.0 \times 10^1$ mN, the specimens being ten of the particles, the compressive test forces being obtained according to a method specified in JIS Z8844 when a loading speed is 0.4462 mN/sec, and
   an alkali metal content is at most 50 ppm.

3. A porous spherical silica **characterized in that**

   a 50% cumulative diameter (D50) of volume based particle size distribution measured by a laser diffraction scattering method ranges from 2 to 200 $\mu$m,
   a ratio (D10/D90) of a 10% cumulative diameter (D10) of the distribution to a 90% cumulative diameter (D90) of the distribution is at least 0.3,
   a pore volume by a BJH method is 0.5 mL/g to 8 mL/g,
   a mode pore radius by a BJH method is 5 nm to 50 nm,
   a specific surface area by a BET method is 100 $m^2$/g to 400 $m^2$/g, and
   an alkali metal content is at most 50 ppm.

4. A polish comprising the porous spherical silica according to any one of claims 1 to 3.

5. A cosmetic comprising the porous spherical silica according to any one of claims 1 to 3.

6. A resin composition comprising the porous spherical silica according to any one of claims 1 to 3.

7. A method of producing a porous spherical silica, the method comprising:

   preparing a W/O emulsion comprising an aqueous phase where a fumed silica is dispersed, and an organic phase including a nonaqueous solvent as a major component;
   heating the emulsion to gelate the aqueous phase to obtain a porous spherical silica dispersion; and
   collecting the generated porous spherical silica from the dispersion.

8. The method according to claim 7, wherein urea is further dissolved in the aqueous phase where the fumed silica is dispersed.

9. The method according to claim 7 or 8, further comprising:

   drying the porous spherical silica collected from the dispersion; and
   optionally, further firing the dried porous spherical silica.

## INTERNATIONAL SEARCH REPORT

| | International application No. |
|---|---|
| | **PCT/JP2022/000754** |

### A. CLASSIFICATION OF SUBJECT MATTER

***C01B 33/18***(2006.01)i
FI:    C01B33/18 Z; C01B33/18 E

According to International Patent Classification (IPC) or to both national classification and IPC

### B. FIELDS SEARCHED

Minimum documentation searched (classification system followed by classification symbols)

C01B33/18

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus (JDreamIII); JST7580 (JDreamIII); JSTChina (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2020-193144 A (NOURYON CHEMICALS INTERNATIONAL B.V.) 03 December 2020 (2020-12-03) | 1-3, 7, 9 |
| | claims 1-5, paragraphs [0048], [0109]-[0116], [0129], [0131] | |
| Y | | 4-6, 8 |
| X | JP 2020-193145 A (NOURYON CHEMICALS INTERNATIONAL B.V.) 03 December 2020 (2020-12-03) | 1-3, 7, 9 |
| | claims 3, 7-8, paragraphs [0053], [0083], [0120] | |
| Y | | 4-6, 8 |
| X | JP 07-196311 A (ENIRICERCHE SPA) 01 August 1995 (1995-08-01) | 7, 9 |
| | claim 1, paragraphs [0004], [0044], example 16 | |
| Y | | 8 |
| Y | WO 2019/131873 A1 (JGC CATALYSTS & CHEMICALS LTD) 04 July 2019 (2019-07-04) | 4-5 |
| | paragraphs [0001]-[0006] | |
| Y | JP 2018-104023 A (NIPPON KASEI CHEM) 05 July 2018 (2018-07-05) | 4-5 |
| | paragraphs [0002], [0029] | |

☑ Further documents are listed in the continuation of Box C.     ☑ See patent family annex.

| | |
|---|---|
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **26 January 2022** | **08 March 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2022/000754**

## C.     DOCUMENTS CONSIDERED TO BE RELEVANT

| Category* | Citation of document, with indication, where appropriate, of the relevant passages | Relevant to claim No. |
|---|---|---|
| Y | JP 2014-210671 A (TOKUYAMA CORP) 13 November 2014 (2014-11-13) paragraphs [0002], [0020] | 5-6 |
| Y | JP 01-230421 A (NIPPON SHOKUBAI KAGAKU KOGYO CO LTD) 13 September 1989 (1989-09-13) p. 1, right column, lines 1-7 | 5-6 |
| Y | JP 2008-137859 A (TAIYO KAGAKU CO LTD) 19 June 2008 (2008-06-19) claims 1-3, paragraph [0017] | 8 |
| Y | WO 2017/038646 A1 (HITACHI CHEMICAL CO LTD) 09 March 2017 (2017-03-09) claims, paragraphs [0115]-[0118], [0129] | 8 |
| P, X | JP 2021-187692 A (TOKUYAMA CORP) 13 December 2021 (2021-12-13) claims 1-8, paragraphs [0021], [0047], [0054], [0066]-[0067], [0078], examples | 7, 9 |
| P, A | | 1-6, 8 |

Form PCT/ISA/210 (second sheet) (January 2015)

**INTERNATIONAL SEARCH REPORT**
**Information on patent family members**

International application No.

**PCT/JP2022/000754**

| Patent document cited in search report | | | Publication date (day/month/year) | Patent family member(s) | Publication date (day/month/year) |
|---|---|---|---|---|---|
| JP | 2020-193144 | A | 03 December 2020 | US 2020/0376464 A1<br>claims 1-5, paragraphs [0051],<br>[0110]-[0117], [0130], [0132]<br>EP 3744684 A1 | |
| JP | 2020-193145 | A | 03 December 2020 | US 2020/0377373 A1<br>claims 3, 7-8, paragraphs<br>[0057], [0086], [0123]<br>EP 3744683 A1 | |
| JP | 07-196311 | A | 01 August 1995 | US 6103209 A<br>claim 1, paragraphs [0004],<br>[0044], example 16<br>EP 653378 A1 | |
| WO | 2019/131873 | A1 | 04 July 2019 | US 2021/0094835 A1<br>paragraphs [0001]-[0010]<br>JP 2020-73436 A | |
| JP | 2018-104023 | A | 05 July 2018 | (Family: none) | |
| JP | 2014-210671 | A | 13 November 2014 | (Family: none) | |
| JP | 01-230421 | A | 13 September 1989 | (Family: none) | |
| JP | 2008-137859 | A | 19 June 2008 | (Family: none) | |
| WO | 2017/038646 | A1 | 09 March 2017 | JP 2018-87136 A<br>claims, paragraphs [0115]-[011<br>8], [0129] | |
| JP | 2021-187692 | A | 13 December 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004101139 A1
- WO 2012057086 A1
- WO 2019131873 A1

**Non-patent literature cited in the description**

- **BARRETT, E. P. ; JOYNER, L. G. ; HALENDA, P. P.** *J. Am. Chem. Soc.,* 1951, vol. 73, 373